Europäisches Patentamt

European Patent Office

Office européen des brevets

(19)

(11) Publication number: **0 199 367**
**B1**

(12) **EUROPEAN PATENT SPECIFICATION**

(45) Date of publication of patent specification: 03.01.90

(21) Application number: 86105754.5

(22) Date of filing: 25.04.86

(51) Int. Cl.⁵: **G 01 N 33/545,**
G 01 N 33/546, G 01 N 33/76,
G 01 N 33/78

(54) Reagent for detecting antigen-antibody reactions.

(30) Priority: 25.04.85 JP 89777/85

(43) Date of publication of application:
29.10.86 Bulletin 86/44

(45) Publication of the grant of the patent:
03.01.90 Bulletin 90/01

(84) Designated Contracting States:
DE FR GB

(56) References cited:
EP-A-0 073 611
AT-B- 351 174

(73) Proprietor: DAIKIN INDUSTRIES, LIMITED
Shin-Hankyu Building 12/39, Umeda 1-chome
Kita-ku
Osaka-shi Osaka-fu (JP)

(72) Inventor: Sugimura, Mutsuyuki
21/21, Hitotsuya 2-chome
Settsu-shi Osaka-fu (JP)
Inventor: Koizumi, Shun
31/6, Yuyamadai 2-chome
Kawanishi-shi Hyogo-ken (JP)
Inventor: Shimizu, Tetsuo
27/104, Shinkoriyama 2-chome
Ibaraki-shi Osaka-fu (JP)

(74) Representative: Barz, Peter, Dr. et al
Patentanwälte Dipl.-Ing. G. Dannenberg Dr. P.
Weinhold, Dr. D. Gudel Dipl.-Ing. S. Schubert, Dr.
P. Barz Siegfriedstrasse 8
D-8000 München 40 (DE)

EP 0 199 367 B1

## Description

The present invention relates to a reagent for detecting an antigen-antibody reaction which is useful for immunoserologic diagnosis.

The method of immunoserologic diagnosis is widely used in the field of clinical tests because of its convenience and rapidity. With this method, a latex having an antigen or antibody adsorbed thereby is used for detecting the corresponding antibody or antigen in the serum by the coagulation reaction of the latex involving an antigen-antibody reaction.

The known latices useful for this purpose include one comprising polystyrene or a copolymer of styrene and styrenesulfonic acid, acrylic acid, acrylate, methacrylic acid, methacrylate, acetonitrile or the like. For the determination of an antigen or antibody with use of the latex, the coagulation reaction is conducted on a black plate, followed by visual observation, or a spectroscopic quantitative method is resorted to using a spectrophotometer.

Polymers of hydrocarbons such as polystyrene have a high refractive index and differ greatly from water in refractive index, so that latices of such polymers themselves have high turbidity. Accordingly the spectroscopic quantitative method resorting to a coagulation reaction with use of the latex is likely to encounter difficulty in recognizing variations in turbidity and therefore in establishing correlation between the concentration and the turbidity. The result obtained by the quantitative method is consequently not fully reliable.

The object of the present invention is to provide an antigen-antibody reaction detecting reagent for readily quantitatively determining a substance by a spectroscopic method.

The present invention provides a reagent for detecting an antigen-antibody reaction in an agglutination (coagulation) method in the form of a latex comprising as its dispersed substance a fluorine-containing polymer other than poly(tetrafluoroethylene) with a refractive index of up to 1.42 and having a protein adsorbed thereon.

We have found that a fluorine-containing polymer latex has high transparency and excellent properties for use as an antigen-antibody reaction detecting reagent.

Examples of fluorine-containing polymers useful for the fluorine-containing polymer latex of the present invention are homopolymers or copolymers of fluoroolefins such as tetrafluoroethylene, hexafluoropropylene, trifluoroethylene, chlorotrifluoroethylene, vinylidene fluoride, vinyl fluoride and fluoroalkyl(meth)acrylate (exclusive of polytetrafluoroethylene), copolymers of such fluoroolefins and at least one of olefin and fluoroalkyl vinyl ether. Examples of olefins are ethylene, propene and, fluoroalkylethylene. These polymers are up to 1.42, preferably up to 1.38, in refractive index. When the coagulation reaction of a latex is to be checked spectroscopically, it is desired that the latex have high transparency. To obtain a latex of high transparency, the substance to be dispersed must be a polymer the refractive index of which is not greatly different from that of water (1.33). We have found that the above-mentioned fluorine-containing polymers are lower than polymers of other hydrocarbons in refractive index and afford latices having high transparency.

The fluorine-containing polymer latex to be used in this invention is an aqueous dispersion obtained by subjecting such fluorine-containing olefin(s) to emulsion polymerization or emulsion copolymerization in an aqueous medium. The latex particles are suitably 0.01 to 1 $\mu$m, preferably 0.03 to 0.3 $\mu$m, in mean size. When a polymer, such as polyvinylidene fluoride, having a relatively high refractive index (1.42) is desirable or when a stable latex is required, the latex particles preferably have a small mean size, e.g. 0.03 to 0.1 $\mu$m.

While the latex contains an emulsifier and other additives which are added for the preparation of the latex, it is generally desirable to remove these additives by dialysis or other procedure. Stabilizers, buffers, etc. are thereafter added to the latex for the preparation of the present reagent.

The method of causing the latex to absorb a protein serving as an antigen (or antibody) will now be described. Although adsorption can be accomplished usually by mixing the latex with an aqueous solution of the protein serving as an antigen (or antibody), the following adsorption method is desirable. The protein as an antigen (or antibody) is dissolved in an $NaHCO_3$ buffer having a pH of 7 to 9 to a protein concentration of 0.001 to 1%. The solution is admixed with the latex, and the mixture is allowed to stand at 20 to 37°C for 1 to 2 hours, whereby the protein is adsorbed by the latex particles. On standing, the latex particles settle. The supernatant is removed. The same $NaHCO_3$ buffer as above is admixed with the remaining liquid containing the sediment, and the supernatant is removed. This procedure is repeated three times, whereby a major portion of the protein not adsorbed by the latex can be removed. Such a separating procedure is possible because the fluorine-containing polymer latex has a great specific gravity, whereas the latex of polystyrene or like hydrocarbon conventionally used and having a small specific gravity requires the use of a centrifugal separator for the same purpose. In this case, the latex often coagulates to such an extent that the particles will not be smoothly redispersible. It is also a feature of the present invention that use of the fluorine-containing polymer latex eliminates the need for the centrifugal separation.

The above method causes the latex to adsorb the antigen (or antibody), affording a reagent for detecting an antigen-antibody reaction.

Examples of proteins to be adsorbed by the latex according to the invention are gamma globulin, choriogonadotropin, thyroglobulin, antifibrinogen antibody and anti-gamma globulin antibody.

The antigen-antibody reaction can be detected using the reagent of the present invention by admixing with the liquid to be checked, the present reagent adsorbing an antigen (or antibody) by the above method and detecting the resulting coagulation reaction. When the liquid contains the corresponding antibody (or antigen), a coagulation reaction occurs owing to an antigen-antibody reaction. Since the coagulation reaction is detectable as a variation in the turbidity of the mixture of reagent and liquid to be checked, the turbidity is measured by a spectrophotometer in terms of absorbance at a specified wavelength. Consequently, the antibody (or antigen) in the liquid can be quantitatively determined from an increase in the absorbance. In practice, the quantitative determination can be conducted in the following manner.

First, a standard specimen containing a specified amount of an antibody (or antigen) is diluted to varying concentrations to prepare standard specimen dilutions. A reagent of the invention adsorbing the corresponding antigen (or antibody) is admixed with each of the dilutions for reaction. The reaction mixture is placed into a cell having an optical path length of 10 mm and irradiated with light of specified wavelength, and the absorbance is measured using a spectrophotometer. Based on the result thus obtained, a calibration curve is prepared which shows the relation between the amount (concentration) of the antibody (or antigen) and the absorbance.

Next, the reagent of the invention adsorbing the antigen (or antibody) is admixed with the liquid to be checked, and the absorbance of the reaction mixture is measured in the same manner as above. The antibody (or antigen) in the liquid can be quantitatively determined from the absorbance with reference to the calibration curve.

The present invention will be described in greater detail with reference to the following examples and comparative example.

Example 1

Into a three-liter glass-lined autoclave having an anchor type stirrer and temperature adjusting means were placed 1.5 liters of deionized and deoxidized water, 60 g of paraffin wax (m.p. 56°C) and 9 g of $H(CF_2CF_2)_4COONH_4$ serving as a dispersant, and the air within the autoclave was replaced by $N_2$ gas several times. Hexafluoropropene was thereafter injected into the autoclave to a pressure of 0.739 MPa (6.5 kg/cm²G). In the meantime, the internal temperature of the autoclave was adjusted to 80°C. The internal pressure was increased to 0.886 MPa (8.0 kg/cm²G) with tetrafluoroethylene, and the stirrer was set to a speed of 300 r.p.m. Subsequently, 15 cc of an aqueous solution containing 1 g of ammonium persulfate was added to the mixture to initiate a reaction. During the reaction, tetrafluoroethylene was continuously supplied to the reaction system to maintain the internal pressure at 0.886 MPa (8.0 kg/cm²G), while hexafluoropropene was intermittently supplied to the system to maintain the mixture at a hexafluoropropene concentration of 65 to 75 mole%. A 0.2 g quantity of ammonium persulfate was further added at an interval of 4 hours.

Thirty-six hours after the start of the reaction, the stirrer was stopped, and the reaction was terminated upon releasing the monomers. A copolymer latex having a polymer concentration of 30.1 wt.% was obtained. The polymer obtained was made into a film, which was checked by Abbe's refractometer (at a temperature of 20°C using D lamp) to find that the polymer had a refractive index of 1.34.

The latex was purified by ultrafiltration. One part by volume of the purified latex and one part by volume of 1 wt.% human gamma globulin were admixed with 8 parts by volume of $NaHCO_3$ buffer (0.5 M NaCl, 0.1 M $NaHCO_3$, pH 8.3). The mixture was allowed to stand at room temperature for one hour. The procedure of replacing the supernatant by the same $NaHCO_3$ buffer as above was repeated three times to remove the unadsorbed human gamma globulin. The product was finally adjusted to a latex concentration of 1 wt.%.

A 2 cc quantity of the reagent thus obtained and adsorbing human gamma globulin was diluted twentyfold. The dilution was placed into a cell having an optical path length of 10 mm and then checked for absorbance at a wavelength of 400 nm by a spectrophotometer (Model 556, product of Hitachi, Ltd.). The absorbance value was 0.05. A 2 cc quantity of the reagent and 0.1 cc of serum containing a rheumatoid factor (rheumatoid factor detecting reagent RAS reference positive serum, product of Nippon Lyophilization Lab. Co., Ltd.) were mixed together, and the mixture was diluted twentyfold. The absorbance of the dilution similarly measured was 0.3. As a control test, the reagent (2 cc) and 0.1 cc of serum free from the rheumatoid factor were mixed together and diluted twentyfold. The absorbance of the dilution similarly measured was 0.06.

These results indicate that the presence of the rheumatoid factor (antibody) in the liquid to be tested results in a markedly increased absorbance and that the absence of the factor produces little or no increase in the absorbance.

Example 2

A 2 cc quantity of a latex of hexafluoropropene-tetrafluoroethylene copolymer (hereinafter referred to as "FEP latex") having a concentration of 10 wt.% and prepared in the same manner as in Example 1 was admixed with 8 cc of a solution prepared by dissolving 40 mg of human gamma globulin in an $NaHCO_3$ buffer (0.15 M NaCl, 0.1 M $NaHCO_3$, pH 8.5). The mixture was stirred at 37°C for 2 hours to cause the FEP latex particles to adsorb human gamma globulin. Next, 30 cc of the same $NaHCO_3$ buffer as above which further contained 1% of sucrose, 0.2 M of choline chloride and 0.1% of sodium azide was added to the latex as a dispersion stabilizer. The mixture was thereafter allowed to stand, and the procedure of replacing the supernatant by the $NaHCO_3$

buffer was repeated three times to remove the unadsorbed human gamma globulin. The resulting mixture was finally adjusted to an FEP latex concentration of 1.2 wt.% with the NaHCO$_3$ buffer.

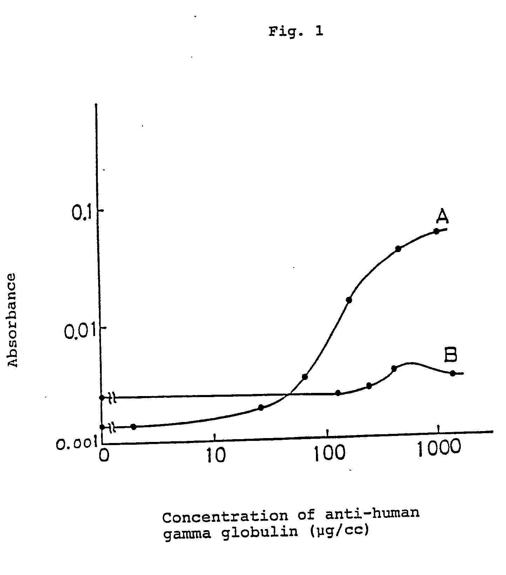

Portions (1.5 cc) of the reagent adsorbing human gamma globulin were admixed with 1.5 cc quantities of anti-human gamma globulin of varying concentrations. The absorbance of the mixtures was measured at a wavelength of 600 nm by the spectrophotometer. The accompanying Fig. 1 shows the relation thus established between the absorbance, plotted as ordinate, and the anti-human gamma globulin concentration, plotted as abscissa.

Comparative Example 1

Into a four-necked separable flask having a capacity of 500 cc, an anchor type stirrer and temperature adjusting means were placed 200 cc of deionized water, 50 g of styrene and 10 cc of an aqueous solution of 0.5 g of styrenesulfonic acid. The stirrer was driven at a speed of 300 r.p.m., the internal temperature of the flask was maintained at 70°C, and the air in the flask was continuously replaced by N$_2$. In this state, 10 cc of an aqueous solution of 0.3 g of ammonium persulfate was added to the mixture to initiate a polymerization reaction. Twelve hours after the start of the reaction, the stirring and introduction of N$_2$ were discontinued to terminate the reaction. The polystyrene latex obtained was 15 wt.% in concentration and 0.21 μm in mean particle size.

Using the polystyrene in place of the FEP latex of Example 2, the relation between the absorbance and the anti-human gamma globulin concentration was determined as shown in the accompanying drawing by the same method as in Example 2 with the exception of using a centrifugal separator (at an increased pressure of 2,65 MPa (26,000 kg/cm$^2$G)) for removing the unadsorbed human gamma globulin and finally adjusting the polystyrene latex concentration to 0.03 wt.%. The polystyrene latex concentration was made lower than the FEP latex concentration (1.2 wt.%) to obtain a comparable absorbance in the absence of the antibody.

Fig. 1 shows that Example 2 (FEP latex reagent) resulted in a close correlation between the amount of antibody and the absorbance but that Comparative Example 1 (polystyrene latex reagent) resulted in a slight correlation therebetween. In particular, the antibody, when present at a very low concentration, produces little or no increase in the absorbance.

The fluorine-containing polymer latex has high transparency, undergoes a distinctive variation in turbidity owing to an antigen-antibody reaction, assures facilitated spectroscopic quantitative determination and possesses excellent properties for use as antigen-antibody reaction detecting reagent.

Fig. 1 shows the results achieved in Example 2 and Comparative Example 1. It is a graph showing the relation between the absorbance and the amount (concentration) of antibody. In the graph, A represents the result achieved by the FEP latex reagent of the invention, and B that achieved by the polystyrene latex reagent of Comparative Example 1.

## Claims

1. A reagent for detecting an antigen-antibody reaction in an agglutination (coagulation) method in the form of a latex comprising as its dispersed substance a fluorine-containing polymer other than poly(tetrafluoroethylene) with a refractive index of up to 1.42 and having a protein adsorbed thereon.

2. A reagent as defined in claim 1 wherein the protein is selected from gamma globulin, choriogonadotropin, thyroglobulin, anti-fibrinogen antibody and anti-gamma globulin antibody.

3. A reagent as defined in claim 1 or 2 wherein the fluorine-containing polymer is up to 1.38 in refractive index.

4. A reagent as defined in any one of claims 1 to 3 wherein the fluorine-containing polymer is a copolymer of tetrafluoroethylene and at least one of hexafluoropropene and perfluoroalkyl vinyl ether.

5. A reagent as defined in any one of claims 1 to 3 wherein the fluorine-containing polymer is a copolymer of vinylidene fluoride and at least one of hexafluoropropene and tetrafluoroethylene.

6. The use of a reagent as defined in any one of claims 1 to 5 for detecting an antigen-antibody reaction in an agglutination (coagulation) method.

## Patentansprüche

1. Reagens zum Nachweis einer Antigen-Antikörper-Reaktion in einem Agglutinations-(Coagulations-)Verfahren in Form eines Latex, umfassend als seine dispergierte Substanz ein von Poly(tetrafluorethylen) verschiedenes, fluorhaltiges Polymer mit einem Brechungsindex von bis zu 1,42, auf dem ein Protein adsorbiert ist.

2. Reagens, wie in Anspruch 1 definiert, worin das Protein ausgewählt ist aus Gamma-Globulin, Choriogonadotropin, Thyroglobulin, Anti-Fibrinogen-Antikörper und Anti-Gamma-Globulin-Antikörper.

3. Reagens, wie in Anspruch 1 oder 2 definiert, worin das Fluor-enthaltende Polymer einen Brechungsindex bis zu 1,38 aufweist.

4. Reagens, wie in irgendeinem der Ansprüche 1 bis 3 definiert, worin das Fluor-enthaltende Polymer ein Copolymer von Tetrafluorethylen und wenigstens einem von Hexafluorpropen und Perfluoralkylvinylether ist.

5. Reagens, wie in irgendeinem der Ansprüche 1 bis 3 definiert, worin das Fluor enthaltende Polymer ein Copolymer von Vinylidenfluorid und wenigstens einem von Hexafluorpropen und Tetrafluorethylen ist.

6. Verwendung eines Reagens, wie in irgendeinem der Ansprüche 1 bis 5 definiert, für den Nachweis einer Antigen-Antikörper-Reaktion in einem Agglutinations-(Coagulations-)Verfahren.

**Revendications**

1. Réactif pour détecter une réaction antigène-anticorps dans une méthode d'agglutination (coagulation), sous la forme d'un latex comprenant, à titre de sa substance dispersée, un polymère fluoré autre que du poly(tétrafluoréthylène), ayant un indice de réfraction d'au plus 1,42 et sur lequel est adsorbée une protéine.

2. Réactif tel que défini dans la revendication 1, dans lequel la protéine est choisie parmi une gamma-globuline, la gonadotropine chorionique, la thyroglobuline, un anticorps anti-fibrinogène et un anticorps anti-gamma-globuline.

3. Réactif tel que défini dans la revendication 1 ou 2, dans lequel l'indice de réfraction du polymère fluoré est d'au plus 1,38.

4. Réactif tel que défini dans l'une quelconque des revendications 1 à 3, dans lequel le polymère fluoré est un copolymère du tétrafluoréthylène et d'au moins l'un de l'hexafluoropropène et d'un éther de vinyle et d'alkyle perfluoré.

5. Réactif tel que défini dans l'une quelconque des revendications 1 à 3, dans lequel le polymère fluoré est un copolymère du fluorure de vinylidène et d'au moins l'un de l'hexafluoropropène et du tétrafluoréthylène.

6. Utilisation d'un réactif tel que défini dans l'une quelconque des revendications 1 à 5 pour la détection d'une réaction antigène-anticorps dans une méthode d'agglutination (coagulation).

Fig. 1

Concentration of anti-human
gamma globulin (µg/cc)